# EUROPÄISCHE PATENTANMELDUNG

(11) **EP 1 031 831 A1**
(43) Veröffentlichungstag der Anmeldung: **30.08.2000**
(21) Anmeldenummer: 00102376.1
(22) Anmeldetag: 04.02.2000
(51) Int. Cl.: G01N 29/12

(54) **Vorrichtung und Verfahren zur Echtheitsprüfung von Edelmetallformkörpern**

(30) Priorität: 27.02.1999 DE 19908620
(71) Anmelder: Degussa-Hüls Aktiengesellschaft, 60287 Frankfurt am Main (DE)
(72) Erfinder: Henning, Carsten, Dr., 60598 Frankfurt/M. (DE); Barbehön, Jörg, Dr., 64285 Darmstadt (DE); Marquis, Heinz, 60388 Frankfurt/M. (DE); Herder, Martin, 63110 Rodgau (DE)

(57) **Zusammenfassung**

Die Erfindung betrifft eine Vorrichtung (1) und ein Verfahren zur Echtheitsprüfung von Edelmetallformkörpern (11), insbesondere von Edelmetallbarren und Edelmetallronden, durch Anregung der Edelmetallformkörper zu Resonanzschwingungen, Messung der Schwingungsfrequenzen und Schwingungsamplituden sowie Vergleich der gemessenen Schwingungen mit Soll- oder Referenzwerten.

Die Vorrichtung (1) beinhaltet
a) eine Einrichtung (5) zur Messung der äußeren Abmessungen des Edelmetallformkörpers;
b) eine Einrichtung (6) zur Schwingungsanregung des Edelmetallformkörpers mit Schall und Messung der Resonanzschwingungen, bei der ein piezoelektrischer Wandler als kombinierter Aktuator und Sensor fungiert;
c) eine Speichereinrichtung, in der die mit den Einrichtungen gemäß a) und b) gemessenen Daten abgelegt werden;
d) eine Speichereinrichtung, in der für bekannte Edelmetallformkörper deren äußere Abmessungen und für deren charakteristische Resonanzschwingungen die Resonanzfrequenzen und Resonanzamplituden der Schwingungsformen bzw. Moden als Referenzdaten abgelegt sind;
e) eine programmgesteuerte Prozessoreinrichtung, die mit den Meßeinrichtungen gemäß a) und b) und den Speichereinrichtungen gemäß c) und d) zusammenwirkt und die einen Vergleich der Meßdaten des zu prüfenden Edelmetallkörpers (11) mit den entsprechenden Referenzdaten vornimmt;
f) ein oder mehrere Einrichtungen (9) zur Eingabe und/oder Auswahl von Daten und/oder zum Abruf von Programmbefehlen;
g) eine Datenausgabeeinrichtung (10), die zumindest eine Information zu Übereinstimmung oder Nichtübereinstimmung anhand des Datenvergleiches abgibt.

## Beschreibung

Die Erfindung betrifft eine Vorrichtung und ein Verfahren zur Echtheitsprüfung von Edelmetallformkörpern, insbesondere von Edelmetallbarren und Ronden, durch Anregung der Edelmetallformkörper zu Resonanzschwingungen, Messung der Schwingungsfrequenzen und Schwingungsamplituden sowie Vergleich der gemessenen Schwingungen mit Soll- oder Referenzwerten. Die erfindungsgemäße Vorrichtung und das Verfahren sind vorzugsweise für die Prüfung von Goldbarren und Goldmünzen vorgesehen, können jedoch grundsätzlich auch auf Formkörper aus anderen Metallen, insbesondere Edelmetallen wie Platin oder Silber, angewendet werden.

Im Handel mit Goldbarren und Goldmünzen ist es üblich, daß Objekte vor dem Ankauf geprüft werden, um sich vor Fälschungen zu schützen, die aus minderwertigen Legierungen oder vollständig oder teilweise aus unedlem Metall hergestellt sind. Hierbei sind insbesondere solche Objekte kritisch, die ganz oder teilweise aus Wolfram bestehen und eventuell nur mit einer dünnen Schicht Gold an der Oberfläche versehen sind. Solche Fälschungen bedeuten für den Käufer einen erheblichen materiellen Schaden und bedeuten für alle potentiellen Käufer einen erheblichen Vertrauensverlust für Gold und andere Edelmetalle als sichere Wertanlage.

Im Handel mit Edelmetallobjekten, etwa bei Banken oder Münzhändlern, kann eine Echtheitsprüfung naturgemäß nur auf Methoden der zerstörungsfreien Materialprüfung basieren.

Zur Prüfung der Echtheit von Edelmetallformkörpern werden bislang Methoden eingesetzt, bei denen über die Ermittlung der äußeren Abmessungen und des Gewichts hinaus zumindest eine materialspezifische physikalische Größe des Prüfkörpers gemessen wird, anhand der der Prüfer dann mittelbar eine Aussage über die Echtheit der Objekte ableiten muß. Die bekannten Verfahren sind dabei jedoch für den Zweck der Echtheitsprüfung im praktischen Handelsalltag mit gewissen Nachteilen verbunden.

Verfahren wie die Röntgenfluoreszenzspektroskopie oder die Funkenspektroskopie verursachen hohe Investitionen für die Prüfgeräte, prüfen die Materialeigenschaften nur an der Oberfläche oder in oberflächennahen Werkstoffbereichen und arbeiten dabei nicht zwingend zerstörungsfrei.

Derartige Verfahren haben demnach die Beschränkung, entweder nur lokal zu prüfen oder einen über das Volumen gemittelten physikalischen Wert zu liefern.

Physikalische Prüfungsverfahren dieser Art haben weiter den Nachteil, daß deren Bedienung speziell geschultes technisch-wissenschaftliches Personal voraussetzt und dieses das erhaltene Ergebnis noch interpretieren muß, um eine Aussage bezüglich der Echtheit abzuleiten. Dies hat zur Folge, daß entsprechende Einrichtungen und entsprechendes Personal nur an wenigen zentralen Stellen, im allgemeinen Prüfstellen oder Analysenlabors, zur Verfügung stehen. Für die Prüfung und den Ankauf eines Objektes bedeutet dies, daß der Prüfkörper zunächst zum entsprechenden Prüflabor geschickt werden muß, um geprüft werden zu können. Anschließend wird das Ergebnis an die ankaufende Stelle übermittelt und das Objekt kann wieder in den Kreislauf zurückgeführt werden. Der entsprechende Vorgang ist somit zeit- und kostenintensiv und birgt das Risiko des Verlustes. Der Verkäufer kann die in Edelmetall gebundene Investition nicht direkt liquide machen und das Objekt steht dem Ankäufer für die Dauer des Prüfvorgangs nicht zum Weiterverkauf zur Verfügung. Als Konsequenz daraus kann es vorkommen, daß Objekte nur unzureichend, etwa mittels einer Sichtprüfung oder einer einfachen Wägung, geprüft werden, was den notwendigen Sicherheitsaspekten nicht hinreichend Rechnung trägt.

Verfahren der Ultraschallprüfung sind vom Grundsatz her technisch einfacher zu realisieren als die vorgenannten Verfahren. Bei diesen wird die Laufzeit einer Ultraschallwelle in einem Prüfkörper gemessen. Aus der so indirekt erhaltenen Schallgeschwindigkeit kann dann auf das Material geschlossen werden. Neben systembedingten und technischen Beschränkungen, die die Umsetzung derartiger Verfahren für die Echtheitsprüfung von Edelmetallformkörpern im praktischen Alltag erschweren, hat die Ultraschallprüfung den Nachteil, daß das Ergebnis durch den Prüfer interpretiert werden muß, um zu einer Aussage über die Materialechtheit zu gelangen, da explizit nur der Zahlenwert einer Laufzeit erhalten wird.

Aus EP 0 113 031 ist eine Vorrichtung zur Echtheitsprüfung von Edelmetallformkörpern bekannt, bei der der Prüfkörper über einen Schwingungsgeber mit Transversalschwingungen beaufschlagt und mit einem Schwingungsaufnehmer die Resonanzfrequenz gemessen wird. Hierbei sind sowohl der Schwingungsgeber als auch der Schwingungsaufnehmer als piezoelektrische Wandler ausgebildet. Aufgrund des komplexen feinwerktechnischen Aufbaus ist diese Vorrichtung empfindlich gegenüber unsachgemäßer Behandlung. Alle wesentlichen Vorgänge des Prüfungsablaufes, wie Justierung des Schwingungsgebers (Aktuators), Ausrichtung des Aufnehmers (Sensors), Auswahl und Durchlauf des zu vermessenden Frequenzbereiches, sind manuell vorzunehmen. Da letztendlich nur Resonanzfrequenzen gemessen werden, ist zur Echtheitsbestimmung ein Abgleich mit in tabellarischer Form vorliegenden Referenzdaten von Originalobjekten erforderlich, den der Prüfer selbst vorzunehmen hat. Für eine eindeutige Aussage ist in jedem Fall eine gesonderte Bestimmung der äußeren Abmessungen sowie sicherheitshalber eine Gewichtsbestimmung des Prüfkörpers erforderlich, da die Schwingungsfrequenzen neben dem materialspezifischen Elastizitätsmodul von den Abmessungen abhängen. Auch diese Daten sind mit tabellarischen Referenzdaten zu vergleichen. Das gesamte Prüfverfahren ist somit arbeits- und zeitaufwendig und in hohem Maße fehleranfällig.

Ziel der vorliegenden Erfindung war es nun, ein sicheres, einfach zu bedienendes, schnelles und robustes Prüfsystem zu entwickeln, das speziell zur Echtheitsprüfung von Edelmetallformkörpern im Handel dient, und welches auch aufgrund seiner Größe, seiner problemlosen Bedienung und des Preises an praktisch allen Stellen für den Ankauf von Edelmetallbarren und Edelmetallmünzen vorgehalten und eingesetzt werden kann. Dieses sollte auf der Methode der Resonanzfrequenzprüfung basieren und war so weiterzuentwickeln, daß die genannten Nachteile beseitigt und insbesondere eine maximale Prüfsicherheit gewährleistet werden.

Erfindungsgemäß wird dieses Ziel erreicht durch eine Vorrichtung zur Echtheitsprüfung von Edelmetallformkörpern, insbesondere von Edelmetallbarren und Edelmetallronden, durch Anregung der Edelmetallformkörper zu Resonanzschwingungen, Messung der Schwingungsfrequenzen und Schwingungsamplituden sowie Vergleich der gemessenen Schwingungen mit Soll- oder Referenzwerten, die dadurch gekennzeichnet ist, daß sie beinhaltet
a)eine Einrichtung zur Messung der äußeren Abmessungen des Edelmetallformkörpers;
b)eine Einrichtung zur Schwingungsanregung des Edelmetallformkörpers mit Schall und Messung der Resonanzschwingungen, bei der ein piezoelektrischer Wandler als kombinierter Aktuator und Sensor fungiert;
c)eine Speichereinrichtung, in der die mit den Einrichtungen gemäß a) und b) gemessenen Daten abgelegt werden;
d)eine Speichereinrichtung, in der für bekannte Edelmetallformkörper deren äußere Abmessungen und für deren charakteristische Resonanzschwingungen die Resonanzfrequenzen und Resonanzamplituden der Schwingungsformen bzw. Moden als Referenzdaten abgelegt sind;
e)eine programmgesteuerte Prozessoreinrichtung, die mit den Meßeinrichtungen gemäß a) und b) und den Speichereinrichtungen gemäß c) und d) zusammenwirkt und die einen Vergleich der Meßdaten des zu prüfenden Edelmetallformkörpers mit den entsprechenden Referenzdaten vornimmt;
f)ein oder mehrere Einrichtungen zur Eingabe und/oder Auswahl von Daten und/oder zum Abruf von Programmbefehlen;
g)eine Datenausgabeeinrichtung, die zumindest eine Information zu Übereinstimmung oder Nichtübereinstimmung anhand des Datenvergleiches abgibt.

Die erfindungsgemäße Vorricht zur Echtheitsprüfung von Edelmetallformkörpern verfügt somit über eine Einrichtung zur Messung der äußeren Abmessungen des Prüfkörpers und eine Einrichtung zur Schwingungsanregung und Messung der Resonanzschwingungen. Damit können alle für eine eindeutige Identifizierung des Prüfkörpers und eine sichere Echtheitsaussage erforderlichen Daten ermittelt werden.

Erstere kann als Meßlehre ausgestaltet sein, deren Backen die Abmessungen des Prüfkörpers abgreifen.

Erfindungsgemäß erfolgt die Schwingungsanregung und Messung der Resonanzschwingung durch einen piezoelektrischen Wandler, der als kombinierter Aktuator und Sensor ausgestaltet ist. Piezoelektrische Wandler, deren zweckgerechte Ausgestaltung und ihr Betrieb sind an sich bekannt.

Weiterhin verfügt die Vorrichtung über eine Speichereinrichtung, in der die gemessenen Daten über die äußeren Abmessungen des Prüfkörpers und über dessen Resonanzschwingungen abgelegt werden, sowie eine weitere Speichereinrichtung, in der die entsprechenden Daten von bekannten Edelmetallformkörpern als Referenzdaten abgelegt sind. Diese Speichereinrichtungen können übliche mikroelektronische Bauelemente sein.

In einer weiterhin vorgesehenen programmgesteuerten Prozessoreinrichtung, die mit den vorgenannten Meß- und Speichereinrichtungen zusammenwirkt, wird ein Vergleich der Meßdaten des zu prüfenden Edelmetallformkörpers mit den Referenzdaten von dem entsprechenden Originaltyp verglichen.

Als Prozessoreinrichtungen kommen übliche mikroelektronische Prozessoren in Frage, die in an sich bekannter Weise programmiert werden. Die Vorrichtung verfügt darüberhinaus über eine oder mehrere Einrichtungen zur Eingabe und/oder Auswahl von Daten und/oder zum Abruf von Programmbefehlen. Diese Eingabeeinrichtung kann etwa in Form einer Tastatur realisiert sein.

Schließlich ist eine Datenausgabeeinrichtung vorgesehen, die zumindest eine Information zur Übereinstimmung oder Nichtübereinstimmung anhand des Datenvergleichs abgibt und somit eine direkte Aussage zur Echtheit des Edelmetallformkörpers trifft. Eine solche Datenausgabeeinrichtung kann etwa in Form von optischen oder akustischen Signalelementen oder in Form einer alphanummerischen und/oder graphischen Anzeigefläche realisiert sein.

In dem erfindungsgemäßen Verfahren zur Echtheitsprüfung von Edelmetallformkörpern werden zunächst in einem ersten Schritt mittels der Meßeinrichtung die äußeren Abmessungen des Prüfkörpers gemessen und in der Speichereinrichtung abgelegt.

In einem zweiten Schritt werden mittels der Einrichtung zur Schwingungsanregung und Messen der Resonanzfrequenz, bei der ein piezoelektrischer Wandler als kombinierter Aktuator und Sensor fungiert, der Prüfkörper mit Schall zu Schwingungen angeregt und dessen Resonanzschwingungen gemessen und in der Speichereinrichtung abgelegt. Diese Schritte laufen programmgesteuert über die mit den Meß- und Speichereinrichtungen zusammenwirkende Prozessoreinrichtung ab.

In einem nächsten Schritt werden durch die programmgesteuerte Prozessoreinrichtung die von dem Prüfkörper ermittelten Meßdaten mit den in der weiteren Speichereinrichtung abgelegten Soll- oder Referenzdaten für den entsprechenden Originaltyp verglichen. Schließlich wird das Ergebnis dieses Vergleiches als Information zu Übereinstimmung oder Nichtübereinstimmung und somit als direkte Aussage über die Echtheit des Edelmetallformkörpers über die Datenausgabeeinrichtung abgegeben.

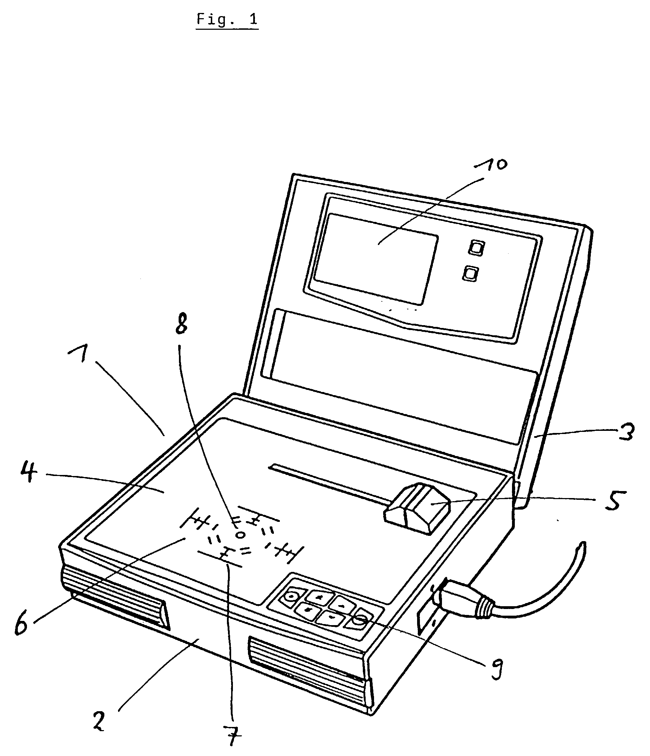

In Fig. 1 ist schematisch eine beispielhafte Ausgestaltung einer erfindungsgemäßen Vorrichtung zur Echtheitsprüfung von Edelmetallformkörpern dargestellt.

Die Vorrichtung (1) weist ein zweiteiliges Gehäuse auf, das aus einem Basisteil (2) und einem auch als Abdeckung fungierenden, klappbarem Oberteil (3) besteht, in denen die Elektronik und Meßmechanik (nicht gezeigt) untergebracht sind. Im aufgeklappten, betriebsbereiten Zustand weist das Basisteil (2) eine horizontale Arbeitsoberfläche (4) auf. Auf dieser Oberfläche ist eine Meßlehre mit programmgesteuert verschiebbaren Meßbacken (5) angeordnet. Mit diesen werden die äußeren Abmessungen des Prüfkörpers abgegriffen. Auf der Oberfläche ist weiterhin die Einrichtung (6) zur Schwingungsanregung und Messung der Resonanzfrequenz des Prüfkörpers angeordnet. Diese weist eine Auflage für den Prüfkörper mit Markierungen (7) zu dessen Zentrierung auf. Die Auflage besteht zweckmäßigerweise aus einem elastischen Material, etwa geschäumtem Kunststoff. In dessen Zentrum befindet sich eine Öffnung (8) durch die ein nadelförmig ausgestalteter, kombinierter Aktuator und Sensor (nicht gezeigt) von unten her mit dem aufgelegten Prüfkörper in dessen Mitte in Kontakt tritt.

Auf der Arbeitsoberfläche befindet sich weiterhin eine Eingabevorrichtung zur Eingabe und Auswahl von Daten und zum Abruf von Programmbefehlen in Form einer Tastatur (9), die etwa als Folientastatur ausgeführt sein kann. Im Gehäuseoberteil (3) befindet sich eine Datenausgabeeinrichtung in Form eines Anzeigefeldes (10), die etwa als Flüssigkristall-Display ausgeführt sein kann.

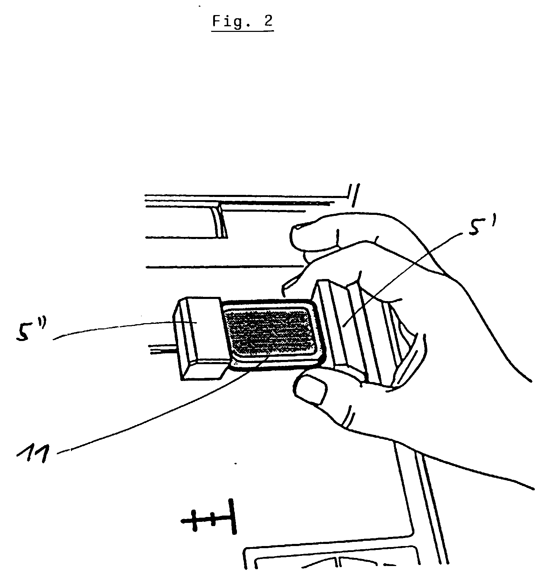

In Fig. 2 ist in einer Ausschnittsdarstellung der erfindungsgemäßen Vorrichtung gemäß Fig. 1 der Vorgang der Messung der Außenabmessungen eines Prüfkörpers (11), hier eines Edelmetallbarrens, dargestellt. Zwischen die geöffneten Meßbacken (5', 5'') der Meßlehre, von denen einer (5') feststehend und der andere (5'') motorisch verschiebbar ist, wird der Barren plaziert. In zwei bzw. drei automatisiert nacheinander ablaufenden Meßvorgängen werden die Außenabmessungen des Prüfkörpers (Durchmesser und Dicke bei Ronden; Länge, Breite und Höhe bei Barren) abgegriffen.

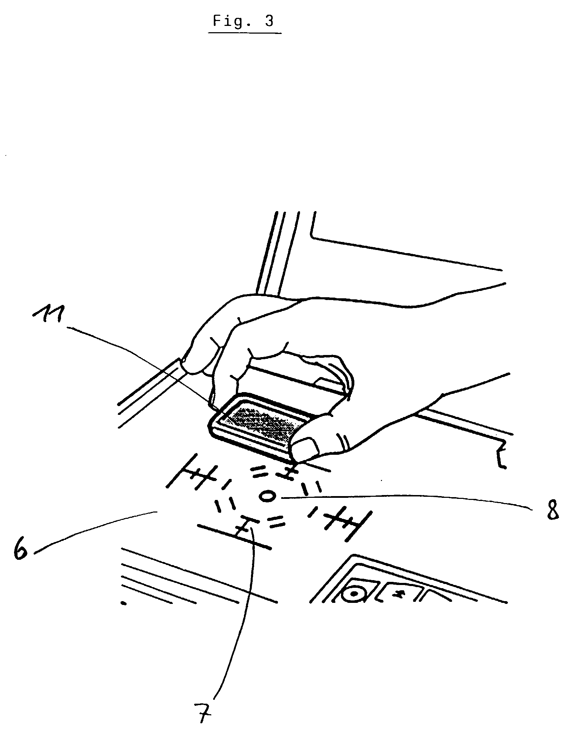

Fig. 3 zeigt ebenfalls in einer Ausschnittsdarstellung den anschließenden Vorgang der Resonanzfrequenzmessung. Der Prüfkörper (11) wird anhand der Marken (7) zentriert auf der Auflage (6) plaziert. In einem automatisiert ablaufenden Vorgang werden der Aktuator/Sensor von unten her durch die Öffnung (8) an den Prüfkörper herangeführt, mit dem er in dessen Mitte in Kontakt tritt, und die Schwingungsanregung und die Frequenzmessung vorgenommen.

Bei der Resonanzfrequenzmessung wird zweckmäßigerweise der gesamte Frequenzbereich der Körperschwingungen durchlaufen und es werden für die charakteristischen Resonanzschwingungen die Frequenzen, Amplituden, Schwingungsformen und Moden erfaßt. Diese werden mit den Soll- bzw. Referenzdaten für entsprechende Originaltypen, die etwa als Datenbank in dem Speicher abgelegt sind, verglichen und die daraus resultierende Echtheitsaussage auf dem Anzeigefeld zur Anzeige gebracht.

Es ist vorteilhaft weil zeitsparend, anstatt den gesamten Frequenzbereich zu durchlaufen, lediglich den oder die engeren Frequenzbereiche zu vermessen, in denen laut der Daten für Originalobjekte die charakteristischen Resonanzfrequenzen liegen.

Es ist bereits ausreichend, bei Übereinstimmung der äußeren Abmessungen, die Echtheitsaussage anhand der Daten zu einer Körperschwingung zu treffen. Für eine maximale Sicherheit ist es jedoch zweckmäßig mindestens zwei, vorzugsweise zwei bis vier Resonanzschwingungen des Prüfobjekts zu vermessen.

In einer vorteilhaften Ausführungsform kann die erfindungsgemäße Vorrichtung mit mehreren piezoelektrischen Wandlern ausgestattet sein, die programmgesteuert mit dem Edelmetallformkörper an verschiedenen Punkten in Kontakt treten. Hierdurch ist es möglich, den Prüfkörper gleichzeitig mit verschiedenen Schwingungen anzuregen und/oder verschiedene Resonanzschwingungen gleichzeitig zu messen.

Vorrichtung und Verfahren gemäß der Erfindung ermöglichen somit eine einfache und schnelle Echtheitsprüfung von Edelmetallformkörpern mit einer direkten Echtheitsaussage. Durch die prozessorgesteuerte, automatisierte Erfassung aller für eine Echtheitsaussage notwendigen Daten und Vergleich mit Referenzdaten wird eine größtmögliche Sicherheit gewährleistet. Der als kombinierter Aktuator und Sensor fungierende piezoelektrische Wandler ermöglicht eine einfache und unempflindliche Bauweise und sichert eine präzise Schwingungsanregung und Resonanzschwingungsmessung.

## Patentansprüche

1. Vorrichtung zur Echtheitsprüfung von Edelmetallformkörpern, insbesondere von Edelmetallbarren und Edelmetallronden, durch Anregung der Edelmetallformkörper zu Resonanzschwingungen, Messung der Schwingungsfrequenzen und Schwingungsamplituden sowie Vergleich der gemessenen Schwingungen mit Soll- oder Referenzwerten,
**dadurch gekennzeichnet,**
daß sie beinhaltet
a)eine Einrichtung zur Messung der äußeren Abmessungen des Edelmetallformkörpers;
b)eine Einrichtung zur Schwingungsanregung des Edelmetallformkörpers mit Schall und Messung der Resonanzschwingungen, bei der ein piezoelektrischer Wandler als kombinierter Aktuator und Sensor fungiert;
c)eine Speichereinrichtung, in der die mit den Einrichtungen gemäß a) und b) gemessenen Daten abgelegt werden;
d)eine Speichereinrichtung, in der für bekannte Edelmetallformkörper deren äußere Abmessungen und für deren charakteristische Resonanzschwingungen die Resonanzfrequenzen und Resonanzamplituden der Schwingungsformen bzw. Moden als Referenzdaten abgelegt sind;
e)eine programmgesteuerte Prozessoreinrichtung, die mit den Meßeinrichtungen gemäß a) und b) und den Speichereinrichtungen gemäß c) und d) zusammenwirkt und die einen Vergleich der Meßdaten des zu prüfenden Edelmetallformkörpers mit den entsprechenden Referenzdaten vornimmt;
f)ein oder mehrere Einrichtungen zur Eingabe und/oder Auswahl von Daten und/oder zum Abruf von Programmbefehlen;
g)eine Datenausgabeeinrichtung, die zumindest eine Information zu Übereinstimmung oder Nichtübereinstimmung anhand des Datenvergleiches abgibt.

2. Vorrichtung nach Anspruch 1,
**dadurch gekennzeichnet,**
daß die Einrichtung gemäß a) als Meßlehre mit programmgesteuert verschiebbaren Meßbacken gestaltet ist, die die äußeren Abmessungen des Edelmetallformkörpers abgreifen.

3. Vorrichtung nach Anspruch 1 oder 2,
**dadurch gekennzeichnet,**
daß die Einrichtung gemäß b) als flächige Auflage aus einem elastischen Material gestaltet ist, durch dessen Zentrum hindurch der piezoelektrische Wandler programmgesteuert mit dem aufgelegten Edelmetallformkörper von unten her in dessen Mitte in Kontakt tritt.

4. Vorrichtung nach den Ansprüchen 1 bis 3,
**dadurch gekennzeichnet,**
daß die Einrichtung gemäß b) mehrere piezoelektrische Wandler aufweist, die programmgesteuert mit dem Edelmetallformkörper an verschiedenen Punkten in Kontakt treten.

5. Verfahren zur Echtheitsprüfung von Edelmetallformkörpern, insbesondere von Edelmetallbarren und Edelmetallronden, durch Anregung der Edelmetallformkörper zu Resonanzschwingungen, Messung der Schwingungsfrequenzen und Schwingungsamplituden sowie Vergleich der gemessenen Schwingungen mit Soll- oder Referenzwerten,
**dadurch gekennzeichnet,**daß
a)in einem ersten Schritt mittels einer Meßeinrichtung die äußeren Abmessungen des Edelmetallformkörpers gemessen werden;
b)in einem zweiten Schritt mittels einer Einrichtung zur Schwingungsanregung und Messung der Resonanzfrequenz, bei der ein piezoelektrischer Wandler als kombinierter Aktuator und Sensor fungiert, der Edelmetallformkörper mit Schall zu Schwingungen angeregt und dessen Resonanzschwingungen gemessen werden;
c)die gemäß a) und b) gemessenen Daten in einer Speichereinrichtung abgelgt und
d)mit in einer Speichereinrichtung abgelegten Soll- oder Referenzdaten, in der für bekannte Edelmetallformkörper deren äußere Abmessungen und für deren charakteristische Resonanzschwingungen die Resonanzfrequenzen und Resonanzamplituden der Schwingungsformen bzw. Moden erfaßt sind, mittels
e) einer programmgesteuerten Prozessoreinrichtung, die mit den Meßeinrichtungen gemäß a) und b) und den Speichereinrichtungen gemäß c) und d) zusammenwirkt, verglichen werden; und
f)mittels einer Datenausgabeeinrichtung eine Information zu Übereinstimmung oder Nichtübereinstimmung abgegeben wird.

6. Verfahren nach Anspruch 5,
**dadurch gekennzeichnet,**
daß mindestens zwei Resonanzschwingungen vermessen werden.

7. Verfahren nach den Ansprüchen 5 oder 6,
**dadurch gekennzeichnet,**
daß bei der Schwingungsanregung ein oder mehrere, nach den für den entsprechenden Edelmetallformkörper abgelegten Referenzdaten ausgewählte Frequenzbereiche durchlaufen werden.

8. Verfahren nach den Ansprüchen 5 bis 8,
**dadurch gekennzeichnet,**
daß mittels mehrerer piezoelektrischer Wandler, die mit dem Edelmetallformkörper an verschiedenen Punkten in Kontakt treten, gleichzeitig mit verschiedenen Schwingungen angeregt und/oder verschiedene Resonanzschwingungen gemessen werden.
